# EUROPEAN PATENT APPLICATION

(11) **EP 3 301 087 A1**
(43) Date of publication of application: **04.04.2018**
(21) Application number: 16191845.3
(22) Date of filing: 30.09.2016
(51) Int. Cl.: C07D 211/56

(54) **PROCESS FOR PREPARING CHIRAL AMINES**

(71) Applicant: DPx Fine Chemicals Austria GmbH & Co KG, 4021, Linz (AT)
(72) Inventor: de Vries, Andre, NL-6228 GZ Maastricht (NL); Lefort, Laurent, NL-6211 JL Maastricht (NL); Verzijl, Gerard, Heerlen (NL)
(74) Representative: Van den Berg, Frans Richard

(57) **Abstract**

The invention pertains to a process for the preparation of an amine having at least two chiral centers from a ketone having a chiral center at the α position and an amine comprising the steps of:
(a) contacting a ketone having a chiral center at the α position and a primary amine thereby forming an imine;
(b) contacting the imine with a reducing agent in the presence of an enantioselective catalyst to form an amine having at least two chiral centers,
wherein step (a) and/or step (b) are conducted under racemization-enhancing conditions, wherein the racemization-enhancing conditions are achieved by addition of an acid and/or by addition of a salt of a primary amine and an acid, which salt is added in addition to or instead of the primary amine, and wherein the reducing agent is hydrogen.

## Description

The present invention relates to processes for preparing amines having at least two chiral centers.

Such processes are known in the art. Lasaletta et al. (Adv. Synth. Catal., 2005, 347, pp. 1917-1920) were the first to report this transformation for α-subsituted cyclic ketones. The reduction was achieved via transfer hydrogenation using the Noyori catalyst and Et₃N-HCOOH as reducing agent. The amount of the Noyori catalyst is too high to be commercially interesting.

List et al. (J. Am. Chem. Soc., 2006, 128, pp. 13074-13075; Ang. Chem. Int. Ed. 2010, 49, pp. 4612-4614; and Synlett, 2007, pp. 2037-2040) developed a similar procedure for aldehydes and ketones based on organocatalysts using a combination of Hantzsch ester and chiral phosphoric acids. The processes are conducted for 72 hours which renders it commercially unattractive.

Kocovsky et al. (Chem. Eur. J. 2008, 14, pp. 8082-8085) prepared β-amino-acids via asymmetric hydrogenation/dymanic kinetic resolution (ARA-DKR) of β-keto esters and nitriles using trichlorosilane as reductant. Trichorosilane is an expensive reducing agent, which further leads to an undesirable by-product.

Rubio-Pérez et al. (Catal. Sci. Technol. 2014, 4, pp. 2626-2630) used asymmetric hydrogenation with chiral palladium catalysts to reduce the in-situ formed imine from α-substituted cyclic ketones with high diastereoselectivities and enantioselectivities. Also Rubio-Pérez uses a high amount of catalyst, which renders the process not cost effective. Additionally, reagents that are commercially not interesting are being used.

The art provides processes with a limited number of substrates and under conditions that are commercially not interesting as e.g. Rubio-Pérez et al disclose hydrogenations with a low substrate over catalyst ratio.

The objective of the present invention is to provide a novel process as well as an improved process to prepare an amine having at least two chiral centers.

The present invention pertains to a process for the preparation of an amine having at least two chiral centers from a ketone having a chiral center at the α position and an amine comprising the steps of:
(a) contacting a ketone having a chiral center at the α position and a primary amine thereby forming an imine;
(b) contacting the imine with a reducing agent in the presence of an enantioselective catalyst to form an amine having at least two chiral centers,
wherein step (a) and/or step (b) are conducted under racemization-enhancing conditions, wherein the racemization-enhancing conditions are achieved by addition of an acid and/or by addition of a salt of a primary amine and an acid, which salt is added in addition to or instead of the primary amine, and wherein the reducing agent is hydrogen.
The process of the invention allows for the preparation of the amine having at least two chiral centers with a good yield and good selectivity. It was found that by conducting the process at least partially under racemization-enhancing conditions, the good yield and selectivity can be reached even at S/C (substrate over catalyst ratio) exceeding 500. Also under these applied conditions the enantioselective catalyst suffers less from deterioration or inactivation. Consequently, the inventive process is suitable for commercial production of amines having at least two chiral centers.
In the context of this application, the wording "racemization-enhancing conditions" refers to conditions that allow for or improve the racemization of the ketone having a chiral center at the α position during step (a) of the process, and/or of the imine prior to or during step (b) of the process. When the racemization rate is sufficiently fast, the process can lead to yields well above the (theoretically expected) 50%, and even exceeding 90%. Such racemization-enhancing conditions can be achieved by addition of an acid in steps (a) and/or (b) and/or by addition of a salt of a primary amine and an acid, which salt is added in addition to or instead of the primary amine. Additionally, the racemization-enhancing conditions can be further improved by conducting steps (a) and/or (b) at a temperature of between 25°C and 120°C. Preferably, the process is conducted at a temperature of between 25°C and 120°C with the use of a salt of the primary amine and an acid.

In one aspect, the process is conducted at a temperature of at least 30°C, preferably at least 40°C and most preferably at least 50°C, and preferably at most 100°C, more preferably at most 90°C and most preferably at most 80°C. The temperature may be different for steps (a) and (b). In such case, the temperature used in step (b) is preferably higher than the temperature used in step (a). In the event that the formation of the imine in step (a) is relatively slow, the temperature in step (a) is preferably higher than the temperature in step (b).

The acid may be any acid that can be suitably used in the present invention. Examples of such acids include formic acid, trifluoroacetic acid, triflic acid, benzoic acid, methane sulfonic acid, p-toluene sulfonic acid, acetic acid, oxalic acid, hydrogen chloride, sulphuric acid and phosphoric acid. The preferred acid is acetic acid. It was found that addition of a strong base, e.g. potassium tert-butoxide (tBUOK), generally leads to mild or strong deactivation of the enantioselective catalyst in step (b) of the process. Hence, no base is added during step (a) and/or (b) of the inventive process. It is noted that the primary amine is generally considered to be a weak base, and exposure of the catalyst to the amine was found not to lead to deactivation.

In one embodiment of the invention, the molar ratio of the acid and the primary amine is at least 0.1, preferably at least 0.5, more preferably at least 0.8 and most preferably at least 1, and generally at most 10, preferably at most 8, more preferably at most 6 and most preferably at most 5.
In one aspect, the acid may be present as solvent or co-solvent.

In a further aspect of the invention, the molar ratio of the ketone having a chiral center and the enantioselective catalyst is generally at least 50. In the context of this application, the said molar ratio is also referred to as "S/C ratio", "S/C" or "substrate-to-catalyst ratio". Preferably, the molar ratio is at least 500, more preferably at least 1000, even more preferably at least 5000 and most preferably at least 10,000, and preferably at most 100,000, more preferably at most 75,000, even more preferably at most 50,000, and most preferably at most 30,000. The racemization-enhancing conditions enable the

catalyst to be more active and/or selective, therewith enabling higher S/C ratios, which in turn renders the process commercially more interesting.

The ketone having a chiral center at the α position may be any ketone having a chiral center at the α position known in the art. The ketone can be racemic or may be enriched with one of the enantiomers. Preferably, the ketone is racemic. The ketone according to the invention may be aliphatic or aromatic. In case of an aliphatic ketone, the ketone may be linear or cyclic. Preferably the ketone is cyclic.
The ketone of the invention may be a ketone having a chiral center according to formula (1): 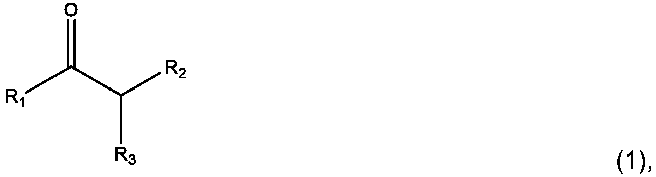 wherein R₁, R₂ and R₃ are separately selected from C₁-_{C12} alkyl, C₁-_{C12} alkenyl, alkaryl and aryl, R₁ and R₃ may optionally form a 5-member or 6-member cyclic structure, optionally comprising one or more heteroatoms, and wherein R₂ is different from R₃. In one embodiment, R₁ and R₃ form a cyclic structure, optionally comprising one or more heteroatoms, and wherein R₂ is different from R₃. The cyclic structure may be substituted. Preferably, the cyclic structure is a 5-, 6-, 7-, or 8-membered ring. More preferably, the cyclic structure is a 5- or 6-membered ring. Even more preferably, R₁ and R₃ form a 6-member cyclic structure, optionally comprising one or more heteroatoms, and wherein R₂ is different from R₃. In another aspect, R₁ and R₃ together form a 6-membered ring comprising one nitrogen in the ring, which is substituted by a benzyl group, and R₂ is methyl. Examples of such cyclic ketones include ketones derived from substituted and non-substituted 5-membered cyclic compounds such as pyrrolidine, tetrahydrofuran (THF), thiolane, imidozoline, pyrazolidine, oxazolidine, thiozolidine, dioxolane, 1,2-dithiolane and 1,3-dithiolane; and substituted and non-substituted 6-membered cyclic compounds such as piperidine, oxane, thiane, piperazine, morpholine, thiomorpholine, 1,2-dioxane, 1,3-dioxane, 1,4-dioxane, 1,2-dithione, 1,3-dithione and 1,4-dithione. Preferred ketones are selected from the group consisting of ketones derived from pyrrolidine, piperidine and piperazine. Examples of such ketones include substituted and non-substituted cyclopentanone, pyrrolidinone, cyclohexanone, piperidinone, tertahydro-4H-pyranone and tetrahydro-4H-thiopyranone. Preferred are ketones selected from the group consisting of ketones derived from pyrollidone and piperidinone. Particularly preferred are ketones that can be used in the preparation of an active pharmaceutical ingredient. An example of such a ketone is a ketone of Formula (2): 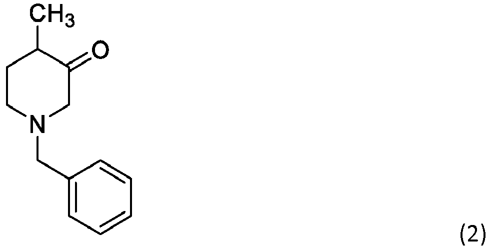 The ketone of Formula (2) can be further converted to its corresponding imine and amine in accordance to the invention, the amine can subsequently be converted to tofacitinib.

The primary amine may be any primary amine known in the art that can suitably be used in the process of the invention. Such primary amines may be aliphatic or aromatic. Preferably the primary amine is aliphatic. Examples of primary amines include alkyl amines such as methyl amine and ethyl amine; ammonia (NH₃) or ammonium (NH₄⁺); alcohol amines such as ethanol amine, alkenyl amines such as allyl amine; phenyl amines such as aniline; and substituted amines thereof. Preferred primary amines are alkyl amines, ammonium, ammonia and alcohol amines. Moreover, further substitution/reaction on the nitrogen is relatively easy. Even more preferred are alkyl amines. Most preferred is methyl amine.
Particularly preferred are primary amines that will result in the N-alkyl group is present in the active pharmaceutical ingredient.

In one aspect, the primary amine can be in the form of a salt with an acid. The salt can be formed before use in step (a) of the inventive process. Alternatively, the salt is formed in situ during step (a). Examples of such acids include formic acid, trifluoroacetic acid, triflic acid, benzoic acid, methane sulfonic acid, p-toluene sulfonic acid, acetic acid, oxalic acid, hydrogen chloride, sulphuric acid and phosphoric acid. The preferred acid is acetic acid.
It is also contemplated to use a combination of a primary amine and a salt of a primary amine and an acid. It is further envisaged to use the combination of a salt of the primary amine and an acid with additional acid.

In step (a) of the process of the invention, the ketone and the primary amine are brought into contact so as to form an imine. In one aspect, step (a) is performed so that at least 80% of the ketone is converted into the imine, preferably at least 85%, more preferably at least 90%, even more preferably at least 95%, even more preferably at least 98% of the ketone is converted into the imine, and most preferably the ketone is completely converted into the imine. After reaching the desired ketone conversion, step (b) is commenced, and the imine is contacted with a reducing agent in the presence of an enantioselective catalyst.
In another embodiment, steps (a) and (b) are combined into one step, which means that the inventive process comprises the step of: contacting a ketone having a chiral center at the α position, a primary amine, a reducing agent and an enantioselective catalyst under racemization-enhancing conditions.

In one embodiment of the invention, the molar ratio of the ketone and the primary amine is at least 0.1, preferably at least 0.5, more preferably at least 0.8 and most preferably at least 1, and generally at most 10, preferably at most 8, more preferably at most 6 and most preferably at most 5.

The reaction mixture of step (a) may further comprise a solvent. Generally a solvent is used which is capable of dissolving the ketone and the primary amine. It is also contemplated to use two or more solvents. Examples of suitable solvents include alcohols such as methanol, ethanol and isopropanol; halogenated alkanes such as dichloro methane and chloroform; carboxylic esters such as methyl acetate and ethyl acetate; trifluoro ethanol, toluene and acetonitrile. Preferred solvents are dichloro methane, ethyl acetate, methanol and isopropanol.

The reaction mixture in step (a) comprises the solvent in an amount of at least 50 % by weight (wt%), based on the total weight of the reaction mixture of step (a). Preferably, the solvent is present in an amount of at least 55 wt%, more preferably at least 60 wt%, even more preferably at least 65 wt% and most preferably at least 70 wt%, and preferably at most 99 wt%, more preferably at most 95 wt%, even more preferably at most 90 wt% and most preferably at most 85 wt%, based on the total weight of the reaction mixture of step (a). The reaction mixture of step (a) includes all ingredients that are necessary to convert the ketone into the imine, i.e. it includes the ketone, the primary amine, the solvent and any other ingredient present (e.g. acid).

When steps (a) and (b) are combined into one step, the reaction mixture comprises the solvent in an amount of at least 20 % by weight (wt%), based on the total weight of the reaction mixture. Preferably, the solvent is present in an amount of at least 25 wt%, more preferably at least 30 wt%, even more preferably at least 35 wt% and most preferably at least 40 wt%, and preferably at most 99 wt%, more preferably at most 95 wt%, even more preferably at most 90 wt% and most preferably at most 85 wt%, based on the total weight of the reaction mixture. In this case, the reaction mixture includes all ingredients that are present in steps (a) and (b) of the inventive process, i.e. the reaction mixture includes the ketone, the primary amine, the reducing agent, the enantioselective catalyst, the solvent and any other ingredient present (e.g. acid).

In step (b) the imine resulting from step (a) is contacted with a reducing agent in the presence of an enantioselective catalyst. The imine may first be isolated from the reaction mixture of step (a) before contacting the imine with the reducing agent and enantioselective catalyst. Preferably, the imine while being in the reaction mixture of step (a) is contacted with the reducing agent and the enantioselective catalyst. In one aspect, step (b) is preferably conducted under racemization-enhancing conditions. The reducing agent is hydrogen. The hydrogen pressure in the process of the invention is generally at least 1 bar, preferably at least 10 bar and most preferably at least 15 bar, and generally at most 200 bar, preferably at most 150 bar and most preferably at most 100 bar.

Step (b) of the process of the invention further comprises an enantioselective catalyst. The enantioselective catalyst can be any catalyst known in the art capable of catalyzing the imine reduction towards one of the enantiomers of the amine having two chiral centers, preferably to the R,R-form or the S,S-form of the amine. The enantioselective catalyst is preferably an organometallic catalyst. In one aspect the catalyst is represented by the formula MLₐX_{b}S_{c}, where M is a transition metal, X is a counter ion and S is a ligand, a ranges from 0.5 to 3 and b and c, each independently, range from 0 to 2, wherein L is a chiral ligand. The organometallic catalyst comprises a transition metal (represented by M) selected from the group consisting of Fe, Co, Ni, Mn, Pd, Pt, Rh, Ru and Ir. Preferably, the organometallic catalyst comprises a transition metal selected from the group consisting of Pd, Pt, Rh, Ru and Ir. The preferred metal is Ir.

The catalyst further comprises at least one ligand (represented by S or L). The ligand can be any ligand suitable for use in the enantioselective catalyst of the invention. The ligand may be a chiral ligand (L) or an achiral ligand (S). Ligand S in the catalyst may be chiral or non-chiral. The achiral ligand may be any achiral ligand known in the art. Examples of such ligands S include olefins such as maleic anhydride or ethylene, dienes such as 1,5-cyclooctadiene, 1,3-butadiene and 2,5-norbornadiene; aromatics such as benzene, hexamethyl benzene, cymene and cumene; and eta-5 coordinated cyclopentadienyl ligands such as cyclopentadienyl and pentamethyl cyclopentadienyl; diamines such as 1,2-diaminoethane. Examples of chiral ligands S include (R,R)-1,2-cyclohexanediamine, (S,S)-1,2-diphenyl-1,2-diaminoethane, (S,S)-1,2-dicyclohexyl-1,2-diaminoethane and (S)-1,1-bis(p-methoxyphenyl)-1,2-propanediamine.

The catalyst preferably comprises at least one chiral ligand L. In one aspect, the chiral ligand contains phosphorous. Suitable ligands can be found in WO 02/04466, WO 2004/35208 and WO 2006/069617, which are hereby incorporated by reference. Example of suitable chiral ligands include biaryl bisphosphine ligands, bisphsopholane ligands derived from DuPhos and BPE, biphosphine ligands derived from DIOP, ferrocene-based biphosphine ligands, P-chiral biphosphine ligands, biphosphinite ligands, biphosphonite ligands, biphosphite ligands, chelating aminophosphine, amidophosphine and phosphoramidites, monophosphorous ligands and N and P-containing ligands. Preferred chiral ligands are selected from the group consisting of MeO-BiPheP, Naud ligand with an oxazoline moiety, Taniaphos and NorPhos. The most preferred ligand is MeO-BiPheP. A particularly preferred MeO-BiPheP ligand is 2,2'-Bis[di(3,5-di-t-butyl-4-methoxyphenyl)phosphino]-6,6'-dimethoxy-1,1'-biphenyl.

The catalyst of the invention further comprises a counter ion (represented by X). The counter ion can be any counter ion known in the art. Examples of suitable counter ions include Cl, BR, I, OAc, BF₄, PF₄, ClO₄, p-toluene sulphonate, benzene phosphonate and tetrapentafluorophenylborate. Non-coordinating anions are preferred.

The enantioselective catalyst can be prepared according to processes known in the art. Such preparations are described for example in WO 02/04466, WO 2004/35208 and WO 2006/069617. The catalyst can be preformed or be prepared in situ, i.e. during step (b) of the process of the invention in the presence of all or part of the reactants.

In one aspect, when the enantioselective catalyst comprises iridium, iodium is added to the reaction mixture in step (b). The iodium may be added as iodium and/or as iodine salt e.g. hydrogen iodide, potassium iodide and ammonium iodium salt. In one aspect, the molar ratio of iodium and the enantioselective catalyst comprising iridium is at least 0.1, preferably at least 0.5, more preferably at least 0.8 and most preferably at least 1, and generally at most 10, preferably at most 8, more preferably at most 6 and most preferably at most 5.

The invention further pertains to a process for the preparation of an amine having at least two chiral centers according to Formula (4): 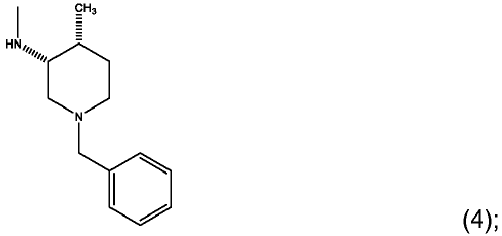 from a ketone having a chiral center at the α position and methyl amine comprising the steps of:
(a) contacting a ketone according to Formula (2): 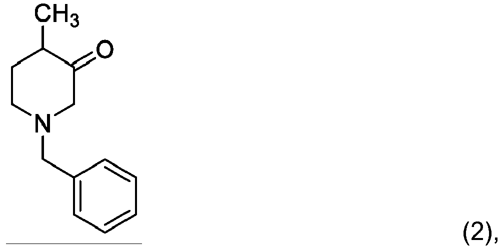 and a primary amine thereby forming an imine of Formula (3): 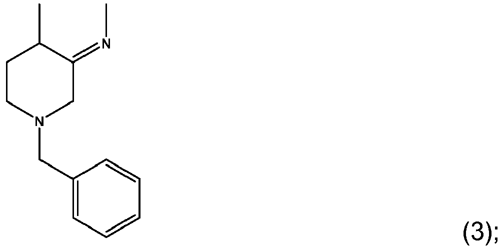
(b) contacting the imine with a reducing agent in the presence of an enantioselective catalyst to form the amine having at least two chiral centers of Formula (4),
   wherein step (a) and/or step (b) are conducted under racemization-enhancing conditions, and wherein the racemization-enhancing conditions are achieved by addition of an acid and/or by addition of a salt of a primary amine and an acid, which salt is added in addition to or instead of the primary amine, and wherein the reducing agent is hydrogen.

The various amounts and reactants as well as the conditions can be chosen as described above.

The invention is exemplified in the following Examples.

### Examples

### Example 1 and 2: Imine formation

The following conversion of ketone 2 with 10 eq methyl amine to imine 3 was performed:

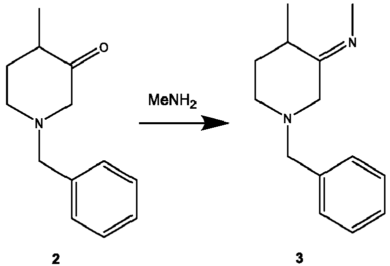

Ketone **2** (20.4mg, 0.1mmol) is placed into a vial and 1 mL of dry isopropanol is added. 33 wt% MeNH₂ in EtOH (124 µL, 1 mmol, 10eq) is added and stirred at 50°C. Analysis is done by GC (HP-5, isotherm at 150°C, 12min). After 17h, the yield of imine 3 is 97% area.

The same reaction was conducted neat - i.e. without adding isopropanol (IPA) - at room temperature. After 5h, the yield of imine **3** is 98% area.

### Comparative Examples 3 to 12: Hydrogenation of preformed imine 3

The imine **3** of Example 2 was converted using hydrogen and an Ir-based enantioselective catalyst into the amine **4.** The cis structures **4a** and **4b** represent the R,R form and the S,S-form of the amine, respectively. The trans structures **4c** and **4d** represent the R,S-form and the S,R-form, respectively.

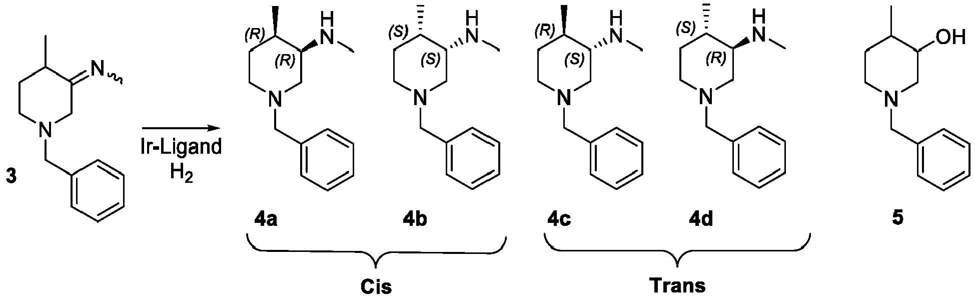

The hydroxyl containing structure **5** represents an undesired side product that is caused by hydrolysis of **3** into the ketone **2** and further hydrogenation of ketone **2.**

The enantioselective catalyst having selectivity towards the amine **4a** selected from ten Ir-based catalysts comprising chiral ligands L1 to L10 according to the corresponding Formulae below were prepared from [Ir(COD)Cl]₂ and ligands L1-L10 in DCM (2h, 40°C).

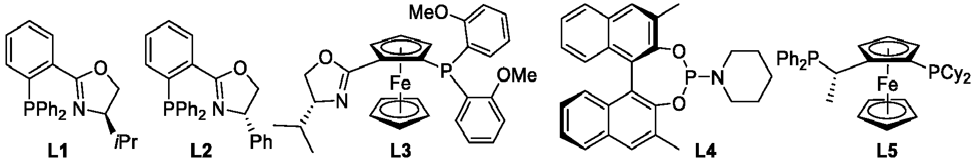

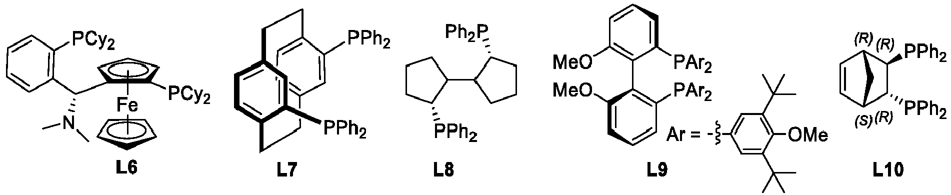

Procedure: The hydrogenation was conducted in the following conditions: Ir (0.001 mmol), I₂ (0 or 0.002mmol), substrate **3** (0.05mmol), IPA = 1mL, 50 bar H₂, R.T., 18h. The yield of the desired diastereomer **4a** was determined by Chiral HPLC.

**Table 1: Yield of amine 4a and 4b (area %):**

| | Comp. Example | **3** | **4** | **5** | **6** | **7** | **8** | **9** | **10** | **11** | **12** |
|---|---|---|---|---|---|---|---|---|---|---|---|
| | Catalysts | **L1** | **L2** | **L3** | **L4** | **L5** | **L6** | **L7** | **L8** | **L9** | **L10** |
| No I2 | Yield 4a | 36 | 30 | 30 | 33 | 21 | 18 | 39 | 37 | 27 | 22 |
| | Yield 4b | 40 | 47 | 42 | 33 | 22 | 24 | 49 | 48 | 18 | 36 |
| 12 | Yield 4a | 22 | 15 | 48 | 16 | 36 | 29 | 35 | 36 | 21 | 33 |
| | Yield 4b | 18 | 20 | 35 | 19 | 43 | 36 | 48 | 33 | 32 | 42 |

As can be seen in this table, no yield of a single enantiomer (either 4a or 4b) is higher than 50% indicating that no racemization takes places.

Comparative Examples 13 to 22: Hydrogenation of preformed imine 3 in presence of a base in order to allow racemization.

The 10 iridium based catalysts tested in examples 3-13 were used in presence of tBuOK to promote racemization.

Procedure: Same catalysts as described above for Comparative Examples 3 to 12 tested with/without I₂.

Hydrogenation conditions: Ir (0.001 mmol), I₂ (0.002mmol), tBuOK (0.005mmol), substrate 3 (0.05mmol), IPA = 1mL, 50 bar H₂, R.T., 18h.

**Table 2: Yield of 4a (area %):**

| | Comp. Example | **13** | **14** | **15** | **16** | **17** | **18** | **19** | **20** | **21** | **22** |
|---|---|---|---|---|---|---|---|---|---|---|---|
| | Catalyst | **L1** | **L2** | **L3** | **L4** | **L5** | **L6** | **L7** | **L8** | **L9** | **L10** |
| No I2 | Yield 4a | 0.3 | 0.4 | 1.2 | 13.6 | 1.2 | 1.0 | 2.4 | 5.8 | 1.9 | 4.0 |
| | Yield 4b | 0.3 | 0.2 | 0.7 | 12.6 | 1.2 | 0.7 | 1.7 | 4.3 | 1.7 | 3.2 |
| 12 | Yield 4a | 0.4 | 0.4 | 1.1 | 28.4 | 2.0 | 0.7 | 21.7 | 4.9 | 4.0 | 15.6 |
| | Yield 4b | 0.4 | 0.4 | 0.8 | 25 | 1.9 | 0.7 | 18.8 | 4.5 | 3.7 | 12.3 |

As can be seen in Table 2, the yields are much lower than in absence of base (see Table 1). Generally, the catalysts have been deactivated by the addition of base. The enantioselectivity in these comparative examples is furthermore lower compared to using of a salt of the primary amine according to the invention, which is corroborated by the comparable yields of enantiomers **4a** and **4b.**

### Examples 23 to 62: In situ formation of imine 3 with the acetate salt of MeNH₂ and Hydrogenation in presence of additional acid.

Procedure: MeNH₃.OAc was formed by slowly adding MeNH₂ (33 wt% EtOH) to a solution of dry acetic acid in toluene. Upon removal of the volatile components, a white hygroscopic salt was obtained. MeNH₃.OAc was used as a reagent to convert ketone **2** into imine **3** in presence of the hydrogenation catalysts, acetic acid, and hydrogen.

### Substrate: Ketone 2 in presence of 4 eq of MeNH₃.OAc with extra CH₃COOH (1.4eq/substrate 2)

Same catalysts as described above tested with/without I₂ in MeOH and in IPA.

Hydrogenation conditions: Ir (0.001 mmol), I2 (0 or 0.002mmol), substrate **2** (0.05mmol), IPA or MeOH = 1 mL, 50 bar H₂, R.T., 18h.

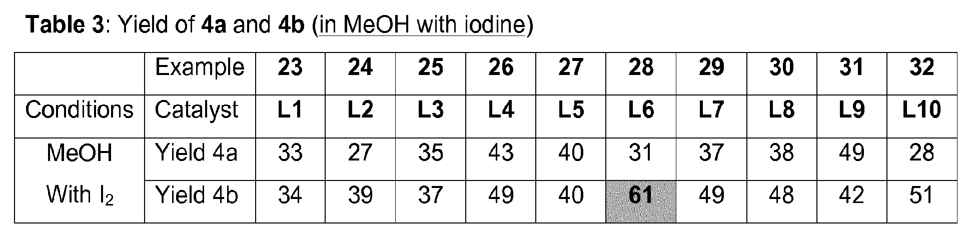

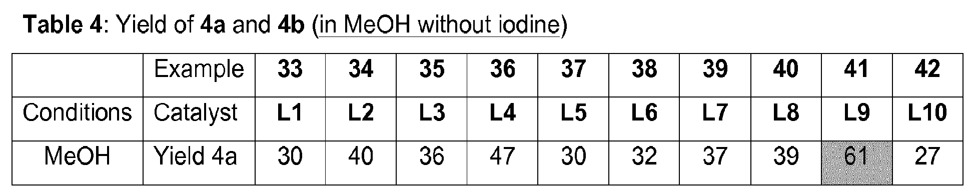

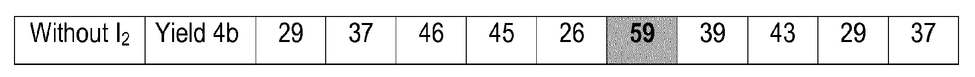

**Table 5: Yield of 4a and 4b (in IPA with iodine)**

| | Example | **43** | **44** | **45** | **46** | **47** | **48** | **49** | **50** | **51** | **52** |
|---|---|---|---|---|---|---|---|---|---|---|---|
| Conditions | Catalyst | **L1** | **L2** | **L3** | **L4** | **L5** | **L6** | **L7** | **L8** | **L9** | **L10** |
| IPA With I₂ | Yield 4a | 24 | 9 | 28 | 20 | 36 | 40 | 44 | 31 | 46 | 41 |
| | Yield 4b | 17 | 11 | 28 | 20 | 30 | 48 | 46 | 36 | 41 | 42 |

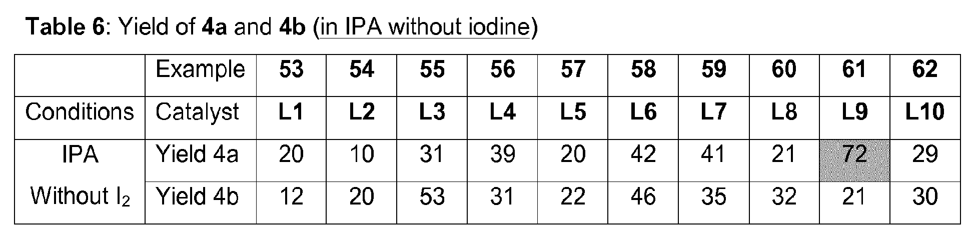

As can be seen in the Table 3 to 6, performing the experiment in presence of an acid drastically improves the yield of a single enantiomer (**4a** or **4b**). In several instances, the yield is above the theoretical yield of 50% indicating that racemization takes place. The best results are obtained with ligand **L9,** the MeO-BiPhep ligand, 2,2'-Bis[di(3,5-di-t-butyl-4-methoxyphenyl)phosphino]-6,6'-dimethoxy-1,1'-biphenyl. Moreover, it can be seen that higher yields are obtained than in Comparative Examples 3 to 12 where no acid was present.

### Examples 63 to 66: In situ formation of imine 3 with the acetate salt of MeNH₂ and Hydrogenation in presence of additional acid with Ir/BiPhep at high substrate to catalyst ratio.

The study was performed solely with Ir/BiPheP catalyst that was prepared from [Ir(COD)Cl]₂ in DCM at 50°C for 1/2h.

### Substrate: Ketone 2 in presence of 4 eq of MeNH₃.OAc with extra CH₃COOH.

Hydrogenation conditions: Ir (0.001 mmol), substrate 2 (0.05mmol), solvent = 1 mL, 50 bar H₂, R.T., 18h.

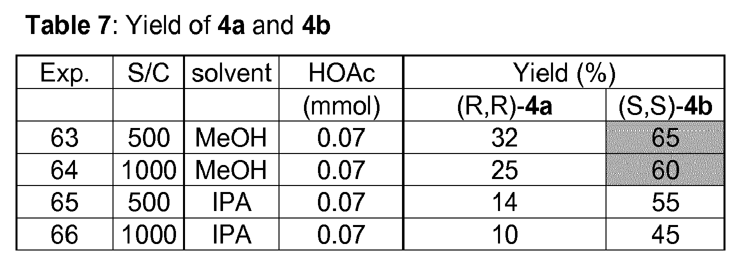

At high substrate to catalyst ratio of 500 and 1000, good yields (above the theoretical yield of 50%) of a single enantiomer are obtained in presence of an acid starting from the racemic ketone 2 and the amine salt.

### Example 67: Synthesis and isolation of 4b on preparative scale with in-situ method and Ir/BiPheP catalyst

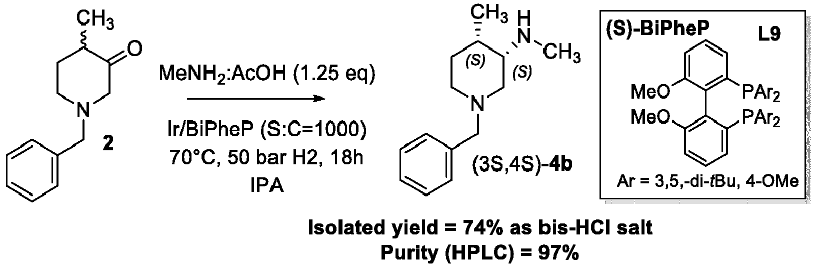

Preparative experiments were performed using only 1.25 eq. N-MeOAc. 10 identical reaction mixtures containing 0.2 mmol starting material 2 were hydrogenated at 70°C, in IPA, under 50 bar of H₂ at S/C=1000 during 18h.

All samples are combined in a round-bottom flask of 100 mL. To the IPA solution, 6N HCl(aq) (1 mL) was added. The solvent IPA is removed under vacuum leaving a yellow solid residue. The residue is dissolved in water (10 mL) and acidified with 6 N HCl (1 mL). The mixture was extracted with toluene (3 x 10 mL). The remaining water layer was basified with NaOH(aq) (32 % (wt)) to pH ∼ 13 in the presence of CH₂Cl₂ (10 mL) while maintaining the temperature to 25°C. The DCM layer was separated after extraction of the product. The remaining water layer was further extracted with DCM (2 x 10 mL). The combined DCM layers were dried over Na2SO4 anhydrous. Removal of DCM under vacuum gives a yellow oily residue (Mass: 406.7mg).

The residue is dissolved in dry acetone (10 mL) and while stirring, 1 N HCl in ether (5 mL) is added. The mixture was allowed to stir for 1 hour. A white solid forms and is filtered off, washed with minimum amounts of acetone. The solid is dried at open air (not hygroscopic) giving the product (**4a** and **4b** as bis-HCl salts) in 498 mg (1.72 mmol, Yield: 86 %) yield. The purity was determined to be 97 % according to GC. The ee was determined to be 72%. The isolated yield of **4b** is 74%.

## Claims

1. A process for the preparation of an amine having at least two chiral centers from a ketone having a chiral center at the α position and an amine comprising the steps of:
(a) contacting a ketone having a chiral center at the α position and a primary amine thereby forming an imine;
(b) contacting the imine with a reducing agent in the presence of an enantioselective catalyst to form an amine having at least two chiral centers,
wherein step (a) and/or step (b) are conducted under racemization-enhancing conditions, wherein the racemization-enhancing conditions are achieved by addition of an acid and/or by addition of a salt of a primary amine and an acid, which salt is added in addition to or instead of the primary amine, and wherein the reducing agent is hydrogen.

2. Process according to claim 1 wherein steps (a) and/or (b) are conducted at a temperature to between 25°C and 120°C.

3. Process according to any one of claims 1 and 2 wherein the molar ratio of the ketone having a chiral center and the enantioselective catalyst is at least 50, preferably at least 500.

4. Process according to any one of the preceding claims wherein the ketone having a chiral center according to formula (1): 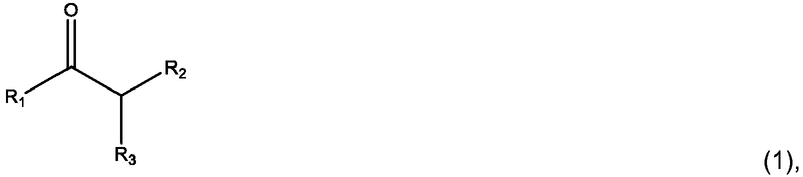 wherein R¹, R² and R³ are separately selected from C₁-_{C12} alkyl, C₁-_{C12} alkenyl, alkaryl and aryl, R¹ and R³ may optionally form a cyclic structure, optionally comprising one or more heteroatoms, and wherein R² is different from R³.

5. Process according to claim 4 wherein R₁ and R₃ together form a 6-membered ring comprising one nitrogen in the ring, which is substituted by a benzyl group, and R₂ is methyl.

6. Process according to any one of the preceding claims wherein the primary amine is an alkyl amine, preferably methyl amine.

7. Process according to any one of the preceding claims wherein the enantioselective catalyst is an organometallic catalyst.

8. Process according to any one of the preceding claims wherein the organometallic catalyst comprises a metal selected from Pd, Pt, Rh, Ru and Ir, preferably Ir.

9. Process according to any one of claims 7 and 8 wherein the catalyst comprises at least one ligand selected from MeO-BiPheP, Naud ligand with an oxazoline moiety, Taniaphos and NorPhos, preferably MeO-BiPheP.

10. Process according to any one of the preceding claims wherein iodine is present in step (b).

11. Process according to any one of the preceding claims wherein the amine having two chiral centers is an R, R-enantiomer.

12. Process according to any one of the preceding claims further converting the amine with two chiral centers to tofacitinib.

13. A process for the preparation of an amine having at least two chiral centers according to Formula (4): 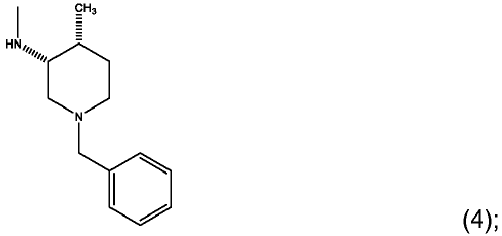 from a ketone having a chiral center at the α position and methyl amine comprising the steps of:
(a) contacting a ketone according to Formula (2): 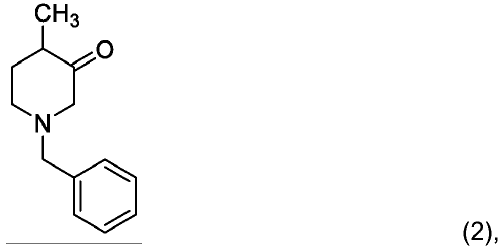 and a primary amine thereby forming an imine of Formula (3): 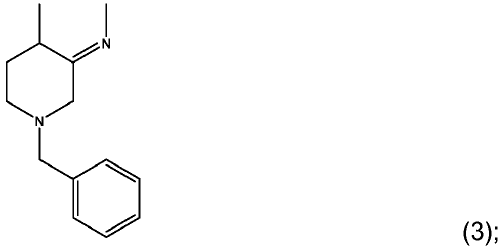
(b) contacting the imine with a reducing agent in the presence of an enantioselective catalyst to form the amine having at least two chiral centers of Formula (4),
wherein step (a) and/or step (b) are conducted under racemization-enhancing conditions, and wherein the racemization-enhancing conditions are achieved by addition of an acid and/or by addition of a salt of a primary amine and an acid, which salt is added in addition to or instead of the primary amine, and wherein the reducing agent is hydrogen.

14. Process according to claim 13 further converting the amine with two chiral centers to tofacitinib.
